# EUROPEAN PATENT SPECIFICATION

(11) **EP 3 025 669 B1**
(45) Date of publication and mention of the grant of the patent: **11.04.2018**
(21) Application number: 14828775.8
(22) Date of filing: 17.06.2014
(51) Int. Cl.: A61B 90/00, B25J 1/02, B25J 9/08, A61B 34/30

(54) **MEDICAL MANIPULATOR**
MEDIZINISCHER MANIPULATOR
MANIPULATEUR MÉDICAL

(30) Priority: 24.07.2013 JP 2013153295
(43) Date of publication of application: 01.06.2016
(73) Proprietor: Olympus Corporation, Hachioji-shi, Tokyo 192-8507 (JP)
(72) Inventor: ISODA, Takumi, Tokyo 151-0072 (JP)
(74) Representative: Gunzelmann, Rainer
(86) International application number: PCT/JP2014/066013
(87) International publication number: WO 2015/012023

(56) References cited:
- WO-A1-2013/062132
- WO-A1-2013/154159
- WO-A2-2011/108840
- WO-A2-2014/005689
- JP-A- H07 213 526
- JP-A- H07 213 526
- JP-A- 2005 168 847
- JP-A- 2009 160 011
- JP-A- 2012 115 553
- JP-A- 2012 235 936
- JP-A- 2013 090 728
- US-A1- 2012 004 648
- US-A1- 2012 143 211

## Description

### TECHNICAL FIELD

The present invention relates to a medical manipulator that is used while inserted through the body cavity to apply treatments to various in-vivo tissues.

### BACKGROUND ART

A medical manipulator system comprising a master manipulator operated by an operator and a slave manipulator for performing treatments on the basis of the actuation of the master manipulator has so far been known as a surgery support system.

In an operation using such a medical manipulator system, an endoscope, a manipulator (or forceps), etc. are inserted in the abdomen or the like of a patient through some small openings formed there. Then, the operator operates a treatment tool mounted on the distal end of the manipulator while viewing endoscopic images on a monitor. Such laparoscopic resection is now expected to have a wider range of applications because there is no need for abdominal operation, resulting in limited burdens on patients and some considerable curtailments in the number

The medical manipulator is often constructed of a proximal unit for supplying driving force to a treatment tool and a distal unit that is attachable to or detachable from the proximal unit and includes a treatment tool at a distal end of a tubular member inserted through the body of a patient.

Referring to such a medical manipulator as described above, for instance, US 7,524,320 discloses that an instrument including an end effector having at least a single degree of freedom at a distal end is attached to a driving assembly by providing one rotary member with a plurality of pins extending from its surface and another rotary member with a plurality of openings in its surface, and fitting the plurality of pins into the plurality of openings. WO 2013/062132 A1, upon which the preamble of claim 1 is based, discloses a medical manipulator including a surgical instrument drive unit having a first input member and a second input member configured to advance and retract in opposite directions to each other and to transmit a driving force in an advance direction, a linear movement conversion unit configured to move the input members forward and rearward, a motor, and a surgical instrument unit having a first transmission member opposite to the first input member and supported to advance and retreat on a connecting end surface, moving in the same direction as of the first input member, and a second transmission member opposite to the second input member and supported to advance and retreat on the connecting end surface, moving in the same direction as of the second input member. US 2012/0143211 A1 refers to a surgical instrument including a use measurement unit configured to acquire a use time or an operation amount of the surgical instrument in response to the use or operation of the surgical instrument.

### SUMMARY OF THE INVENTION

A problem with the system of US 7,524,320 wherein the multiple pins on one rotary member are fitted into the multiple openings in another rotary member is that attachment/ detachment gets cumbersome because of the need of alignment of the two rotary members.

### MEENS FOR SOLVING THE PROBLEMS

To provide a solution to the aforesaid problem, the present invention provides a medical manipulator comprising the features of claim 1. A medical manipulator may include: a distal end side and a proximal end side configured to be attachable to or detachable from each other, comprising:
a distal unit including:
   an end effector on the distal end side,
   a joint assembly located on a proximal end side of said end effector, and
   a first power transmission assembly for transmission in a longitudinal direction of a linear motion force for actuation of said end effector or said joint assembly, and
   a proximal unit including,
   a driver on the proximal end side for generating power for actuating said end effector or said joint assembly, and
   a second power transmission assembly for transmission of power generated from said driver to the distal end side, and detachably mounted on said distal unit at a distal end, wherein:
      said distal unit includes a first linear motion force conversion mechanism linked to said first power transmission assembly to convert power from said second power transmission assembly to a linear motion force upon attachment of said distal unit to said proximal unit.

In the medical manipulator according to the invention, said proximal unit further includes a second linear motion force conversion mechanism for converting rotational power of said driver into a linear motion force, and said second power transmission assembly transmits the linear motion force.

In the medical manipulator according to the invention, said first linear motion force conversion mechanism includes a seesaw member that is rotatable about a rotating shaft, and has a contact portion coming in contact with said second power transmission assembly upon attachment of said distal unit to said proximal unit, wherein said seesaw member is connected to said first power transmission assembly, and as a result of rotation of said seesaw member by power transmitted from said second power transmission assembly, said first power transmission assembly is pushed or pulled in said longitudinal direction.

In the medical manipulator according to the invention, said proximal unit further includes a link member having an abutment portion in abutment on said contact portion of said seesaw member upon linking to a distal end of said second power transmission member for attachment of said distal unit to said proximal unit.

In the medical manipulator according to the invention, said abutment portion and said contact portion are configured in such a way as to have a concave/convex relation in which said both portions are in abutment on each other.

In the medical manipulator according to the invention, said second linear motion force conversion mechanism includes a second rack-and-pinion mechanism for conversion of rotational power of said driver to a linear motion force.

The medical manipulator according to the invention further includes a restriction portion for restricting movement of said second power transmission assembly.

In the medical manipulator according to the invention, said distal unit is encased in such a way as to be watertight.

### ADVANTATES OF THE INVENTION

The medical manipulator according to the invention does not rely upon such a mounting system of fitting a plurality of pins extending from one rotary member into a plurality of openings in another rotary member. The medical manipulator of the invention dispenses with any troublesome mounting because a single mounting is all that is needed to attach the distal unit to the proximal unit.

### BRIEF EXPLANATION OF THE DRAWINGS

Fig. 1 is illustrative in schematic of the construction of the manipulator 100 according to the first embodiment of the invention.
Fig. 2 is illustrative of how the distal unit 300 is mounted in place in the manipulator 100 according to the first embodiment of the invention.
Fig. 3 is illustrative of the actuation of the manipulator 100 according to the first embodiment of the invention.
Fig. 4 is illustrative of the actuation of the manipulator 100 according to the first embodiment of the invention.
Fig. 5 is illustrative of the actuation of the manipulator 100 according to an example.
Fig. 6 is illustrative of the actuation of the manipulator 100 according to the example.
Fig. 7 is illustrative of the actuation of the manipulator 100 according to another example.
Fig. 8 is illustrative of the actuation of the manipulator 100 according to the the example.
Fig. 9 is illustrative of the actuation of the manipulator 100 according to the second embodiment of the invention.
Fig. 10 is illustrative of the actuation of the manipulator 100 according to the second embodiment of the invention.
Fig. 11 is illustrative in schematic of the construction of the manipulator 100 according to the third embodiment of the invention.
Fig. 12 is illustrative of how the distal unit 300 is mounted in place in the manipulator 100 according to the third embodiment of the invention.
Fig. 13 is illustrative of the actuation of the manipulator 100 according to the third embodiment of the invention.
Fig. 14 is illustrative in schematic of the construction of the manipulator 100 according to an example.
Fig. 15 is illustrative of how the distal unit 300 is mounted in place in the manipulator 100 according to the example.
Fig. 16 is illustrative of the actuation of the manipulator 100 according to the example.
Fig. 17 is illustrative in schematic of the construction of the manipulator 100 according to another example.
Fig. 18 is illustrative in schematic of the construction of the manipulator 100 according to another example.
Fig. 19 is illustrative in schematic of the construction of the manipulator 100 according to the fourth embodiment of the invention.
Fig. 20 is illustrative in schematic of the construction of the manipulator 100 according to the fifth embodiment of the invention.
Fig. 21 is illustrative in schematic of the construction of the manipulator 100 according to another example.
Fig. 22 is illustrative in schematic of the construction of the manipulator 100 according to the sixth embodiment of the invention.
Fig. 23 shows an example of the inventive manipulator 100 applied to the master/slave manipulator system 1000.
Fig. 24 shows the endoscope apparatus 110 in the master/slave manipulator system 1000.

### MODES FOR CARRYING OUT THE INVENTION

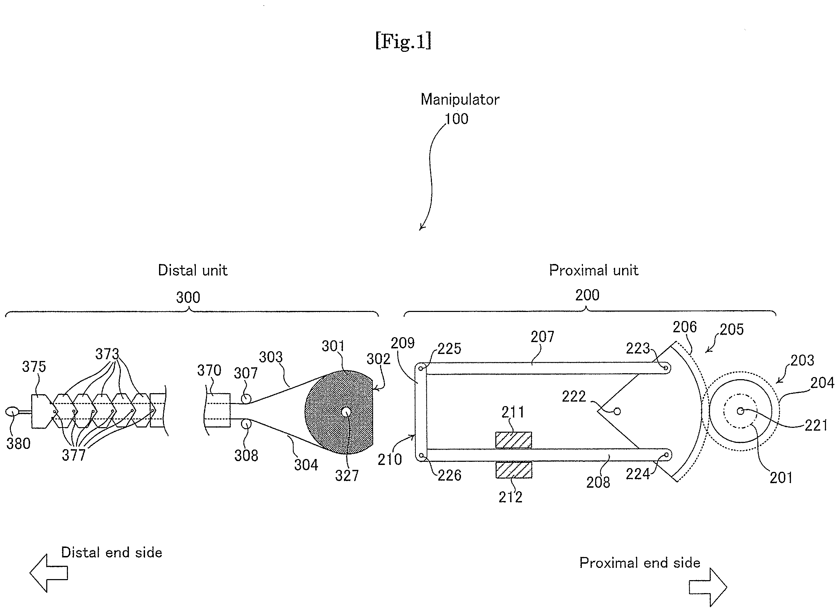

Fig. 1 is a schematic view of the construction of the manipulator 100 according to the first embodiment of the invention. Note here that only the main components will be described in the explanation of the following embodiments, and casings, etc. are not shown in the drawings.

The manipulator 100 comprises a proximal unit 200, and a distal unit 300 to which driving force is supplied from the proximal unit 200 when it is attached to or detached from the proximal unit 200. The proximal unit 200 is assumed to be located on a proximal end side, and an end effector 380 is mounted on the most distal end side of the distal unit 300. The end effector 380 may include a pair of forceps, a retractor, an electric knife, an ultrasonic knife, a laser knife, etc., for in-vivo treating and may further include an imaging unit for in-vivo viewing.

The distal unit 300 is assumed to be used while inserted through a channel (not shown) and a medical trocar (not shown) in a medical endoscope or robot.

A motor 201 (driver) rotationally drives a shaft 221 (for the second linear motion force conversion mechanism) in association with the operation of an operating rod (not shown), etc. for rotation of a gear 203 (for the second linear motion force conversion mechanism).

A gear 205 (for the second linear motion force conversion mechanism) rotates about a shaft 222 (for the second linear motion force conversion mechanism) in association with the rotation of the gear 203.

A pole-form member 207 (for the second power transmission assembly) is mounted at one end on the gear 205 in such a way as to be rotatable about a shaft 223 (for the second power transmission assembly).

The pole-form member 207 is also mounted at the other end on a link member 209 (for the second power transmission assembly) in such a way as to be rotatable about a shaft 225 (for the second power transmission assembly).

A pole-form member 208 (for the second power transmission assembly) is mounted at one end on the gear 205 in such a way as to be rotatable about a shaft 224 (for the second power transmission assembly).

The pole-form member 208 is provided at the other end with the link member 209 in such a way as to be rotatable about a shaft 226 (for the second power transmission assembly).

A first restriction member 211 (for restriction) and a second restriction member 212 (for restriction) are mounted on a stage (not shown) in such a way as to move up or down while a certain distance is maintained between them. The pole-form member 208 is sandwiched between the first 211 and the second restriction member 212 such that its movement is restricted.

While the pole-form member 208 is here used as a pole-form member whose movement is restricted by the first 211 and the second restriction member 212, it is here to be noted that the movement of the pole-form member 207 may also be restricted.

For the reason that the movement of the pole-form member 208 is restricted by the first 211 and the second restriction member 212, both the pole-form members 207 and 208 are displaceable in a parallel state even when the gear 205 is rotated or displaced by the gear 203.

As the pole-form member 207 makes a displacement toward the distal end side in a direction indicated by an action arrow, it causes the pole-form member 208 to make a displacement toward the proximal end side in a direction indicated by an action arrow. Conversely, as the pole-form member 207 makes a displacement toward the proximal end side, it causes the pole-form member 208 to make a displacement toward the distal end side.

The link member 209 has a given thickness vertically with respect to the sheet plane, and the leftmost end portion thereof is in a planar shape as shown. This plane is hereinafter called a link member abutment portion 210 (for the second power transmission assembly; the abutment portion). As the distal unit 300 is mounted on the proximal unit 200, it causes the link member abutment portion 210 to abut on a seesaw member contact portion 302 (for the first linear motion force conversion mechanism) of the seesaw member 301 (for the first linear motion force conversion mechanism; the seesaw portion).

An explanation is then made of the distal unit 300 to or from which the proximal unit 200 constructed as described above is attached or detached.

The seesaw member 301 in the distal unit 300 is configured (D-configuration) as if a disk member were partly cut out: it has a given certain thickness vertically with respect to the sheet plane and the rightmost end portion thereof is in a planar shape as shown. This plane is hereinafter called the seesaw member contact portion 302.

Upon attachment of the distal unit 300 to the proximal unit 200, the link member abutment member 210 of the link member 209 abuts on the seesaw member contact portion 302 of the seesaw member 301 for transmission of driving force from the link member 209 to the seesaw member 301. The seesaw member 301 is rotatable about the seventh shaft 327 (for the first linear motion force conversion mechanism). Note here that a triangle 222-223-224 defined by the respective shafts is congruent with a triangle 225-327-226 defined by the respective shafts.

One end of a wire 303 (for the first power transmission assembly) is fixed to the upper end of the seesaw member 301 (for the first linear motion force conversion mechanism) as by soldering, and the other end of the wire 303 is fixed to the most distal joint-ring 375 (for the joint assembly). The wire 303 is routed around a driven pulley 307 (for the first transmission assembly).

One end of a wire 304 (for the first power transmission assembly) is fixed to the lower end of the seesaw member 301 (for the first linear motion force conversion mechanism) as by soldering, and the other end of the wire 304 is fixed to the most distal joint-ring 375 (for the joint assembly). The wire 304 is routed around a driven pulley 308 (for the first power transmission assembly).

The distal unit 300 includes a cylindrical tubular member 370, a plurality of cylindrical joint rings 373 (for the joint assembly) and the most distal joint ring 375 in order toward the distal end side, and the wires 303 and 304 are inserted into these tubular members.

The cylindrical tubular member 370, multiple cylindrical joint rings 373 and the distal joint ring 375 are coupled together by means of a plurality of rivet-form shaft members 377 (for the joint assembly). The rivet-form shaft members 377 are provided to couple the respective joint rings 373, etc. in a foldable way to form the joints of the manipulator 100.

For instance, the end effector 380 such as an electric knife is mounted on the distal end of the most distal joint ring 375.

An explanation is now made of how the distal unit 300 is mounted in place and how the distal unit 300 is actuated in the manipulator 100 according to the first embodiment of the invention configured as described above.

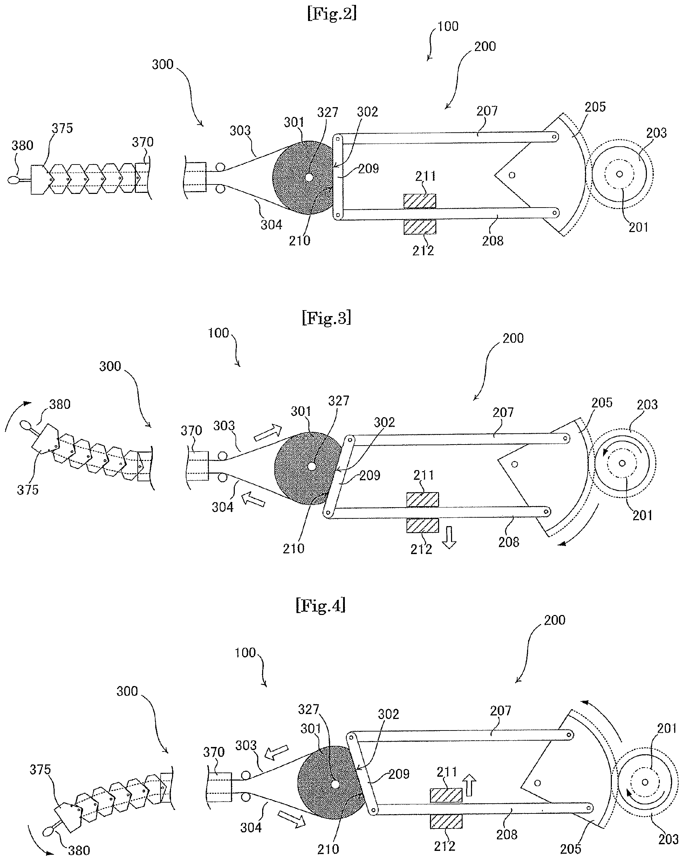

Fig. 2 is illustrative of how the distal unit 300 is mounted in place in the manipulator 100 according to the first embodiment of the invention.

As shown in Fig. 2, upon attachment of the distal unit 300 to the proximal unit 200, the link member abutment portion 210 of the link member 209 abuts on the seesaw member contact portion 302 of the seesaw member 301 for transmission of driving force from the link member 209 to the seesaw member 301. Note here that the embodiment described here is characterized in that the link member 209 abuts on the seesaw member 301 in just one plane alone, facilitating attachment or detachment.

The manipulator 100 of the invention does not rely upon the conventional mounting process of fitting a plurality of pins extending from one rotary member into a plurality of openings formed in another rotary member. According to the manipulator 100 of the invention that makes use of the proximal unit 200 including the link member 209 making a displacement in association with the displacements of the pole-form members 207 and 208 and the distal unit 300 including the seesaw member 301 making a displacement in association with a displacement of the link member 209 for the purpose of ease of mounting/dismounting.

The actuation of the manipulator 100 according to the invention is now explained. Fig. 3 is illustrative of how the manipulator 100 according to the first embodiment of the invention is actuated.

As the motor 201 in the proximal unit 200 is driven to rotate the gear 203 counterclockwise, it causes clockwise rotation of the gear 205 in mesh with the gear 203.

Rotation of the gear 205 causes the pole-form member 207 to make a displacement toward the proximal end side and the pole-form member 208 to make a displacement toward the distal end side. And the first 211 and the second restriction member 212 with the pole-form member 208 in between go down, and the link member abutment portion 210 of the link member 209 turns upward.

The seesaw member contact portion 302 in abutment on the link member abutment portion 210 turns downward and, operatively with this, the seesaw member 301 rotates clockwise about the shaft 327. This in turn causes the wire 303 to be taken up and the wire 304 to be let out with the result that the end effector 380 positioned at the most distal joint ring 375 makes an upward displacement as shown.

Another actuation pattern of the manipulator 100 according to the invention is now explained. Fig. 4 is illustrative of how the manipulator 100 according to the first embodiment of the invention is actuated.

As the motor 201 in the proximal unit 200 is driven to rotate the gear 203 clockwise, it causes counterclockwise rotation of the gear 205 in mesh with the gear 203.

Rotation of the gear 205 causes the pole-form member 207 to make a displacement toward the distal end side and the pole-form member 208 to make a displacement toward the proximal end side. And the first 211 and the second restriction member 212 with the pole-form member 208 in between go up, and the link member abutment portion 210 of the link member 209 turns downward.

The seesaw member contact portion 302 in abutment on the link member abutment portion 210 turns upward and, operatively with this, the seesaw member 301 rotates counterclockwise about the shaft 327. This in turn causes the wire 303 to be let out and the second wire 304 to be taken up with the result that the end effector 380 positioned at the distal joint ring 375 makes a downward displacement as shown.

As described above, the manipulator 100 described here includes the seesaw member 301 that makes an unerring displacement in association with the displacements of the pole-form members 207 and 208 in the proximal unit 200, making sure proper transmission of driving force to the end effector 380.

The manipulator 100 of the invention does not rely upon the conventional mounting process of fitting a plurality of pins extending from one rotary member into a plurality of openings formed in another rotary member. The manipulator 100 according to the invention makes use of the distal unit 300 having the seesaw member 301 making displacements in association with displacements of the pole-form members 207 and 208 in the aforesaid proximal unit 200, contributing more to ease of mounting/dismounting.

An example is now explained. Referring here to the manipulator 100 according to the first embodiment, there is a possibility that the end effector 380 may move although depending on the posture of the link member 209 in the proximal unit 200 upon mounting of the distal unit 300 on the proximal unit 200 after insertion of the distal unit 300 in the body.

To exclude such a possibility, the example described here is configured such that the driving force may be fed to the link member 209 after aligning the posture of the link member 209 in the proximal unit 200 with the posture of the seesaw member 301 in the distal unit 300. More specifically, the example described here includes a mechanism having a function like that of a clutch for making a selection of whether or not the driving force of the motor 201 is transmitted to the pole-form members 207, 208 or the link member 209 (hereinafter called the clutch-like mechanism).

In the example described here, the clutch-like mechanism transmits or cuts off the driving force of the motor 201 by engagement or disengagement of the gears 203 and 205.

The medical manipulator 100 of the invention does not rely upon the conventional mounting process of fitting a plurality of pins extending from one rotary member into a plurality of openings formed in another rotary member. All that is needed for the medical manipulator 100 according to the embodiment described here is just only the coupling of the distal unit 300 to the proximal unit 200, resulting in ease of mounting/dismounting.

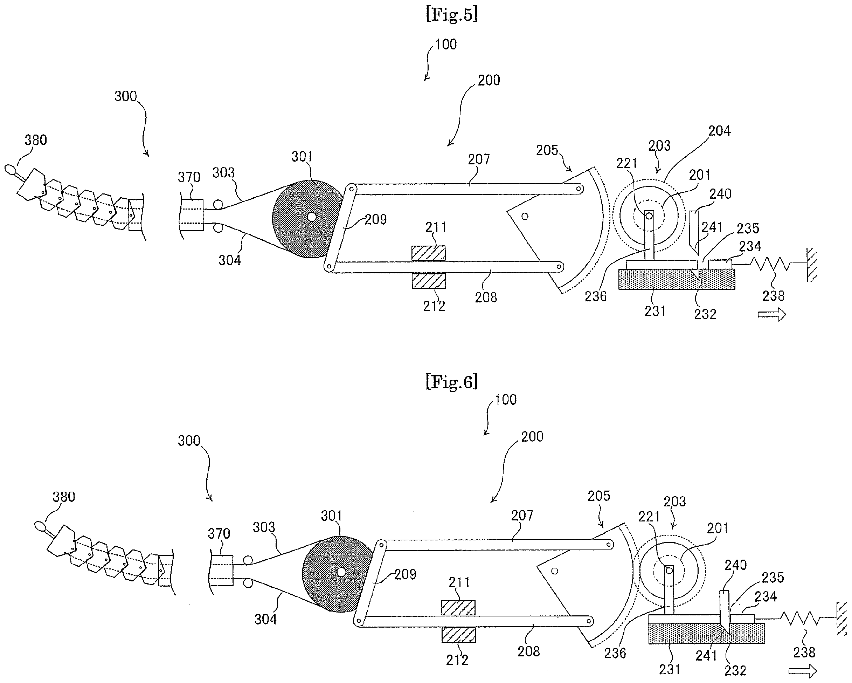

An exemplary configuration of the clutch-like mechanism of the example described here is now explained with reference to Figs. 5 and 6 illustrative of the actuation of the manipulator 100.

Fig. 5 shows that the driving force is disengaged by the clutch-like mechanism: the gear 203 (for the second linear motion force conversion mechanism) is disengaged from the gear 205.

The example described here includes a slide member 234 that slides on a base 231, and an upright portion 236 extending from the slide member 234 is provided with a motor 201 for generation of driving force and a gear 203 that rotates by way of the motor 201.

The slide member 234 is constantly biased by a biasing member 238 toward the proximal end side. The slide member 234 includes a through-hole 235 through which a pawl-form member 240 is insertable, and the base 231 includes a cutout 232 capable of receiving a slant portion 241 of the pawl-form member 240. The pawl-form member 240 is configured by a mechanism (not shown) in such a way as to make a vertical displacement.

The distal unit 300 in the example configured as described above is now explained. As the distal unit 300 is mounted on the proximal unit 200 with the end effector 380 turning upward as shown typically in Fig. 5, it causes the link member 209 on the proximal unit 200 side to be mounted in place following the posture of the seesaw member 301, because that link member 209 is disengaged from the driving source. According to the example described here, there is thus no possibility that the end effector 380 may move.

How the driving force of the motor 201 may be transmitted to the pole-form members 207, 208 or the link member 209 is now explained. Upon a descent of the pawl-form member 240 from the state shown in Fig. 5, it causes the slant portion 241 to urge the inside of the through-hole 235 as it is inserted through the through-hole 235 in the slide member 234, resulting in a displacement of the slide member 234 toward the distal end side. Operatively with this, the gear 203 comes close to the gear 205, and engages the gear 205 in the state of Fig. 6 where the slant portion 241 of the pawl-form member 240 is fully received in the cutout 232 in the base 231. As a result, the driving force of the motor 201 may be transmitted to pole-form members 207, 208 or the link member 209.

In a transition from the driving force transmitted state of Fig. 6 to the driving force disengaged state shown in Fig. 5, the pawl-form member 240 moves up. As the pawl-form member 240 goes up, it causes the slide member 234 to move toward the proximal end side by the biasing member 238. Operatively with this, the gear 203 moves away from the gear 205 to cut off the transmission of driving force of the motor 201 to the pole-form members 207, 208 or the link member 209.

In the example described herein, the transmission of the driving force of the motor 201 may be cut off by a simple actuation involving the ascendant movement of the pawl-form member 240. For instance, an emergent shutoff of the manipulator 100 may simply be carried out only by the ascending movement of the pawl-form member 240 as mentioned above. The example described here has a merit of taking a convenient yet quick step to emergencies.

Another example is now explained. In the manipulator 100 according to the previous example, the clutch-like mechanism performs its function in two modes: engagement and disengagement of the gears 203 and 205. In this example, however, another clutch-like mechanism is relied upon.

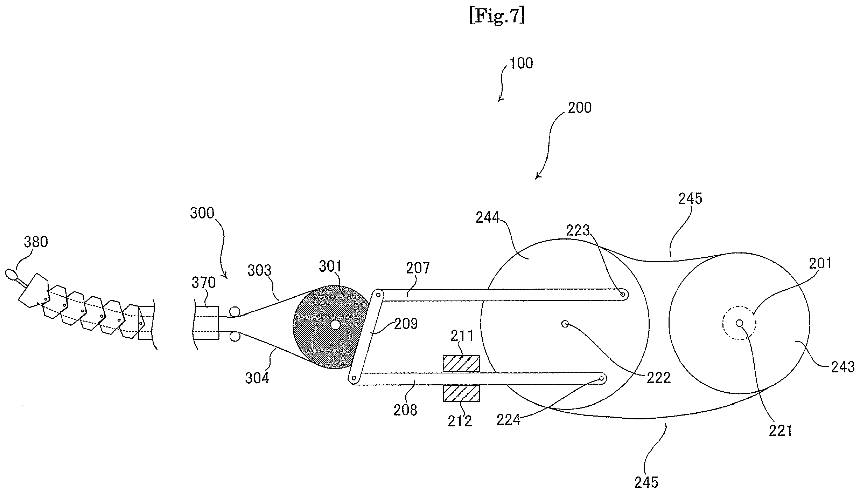

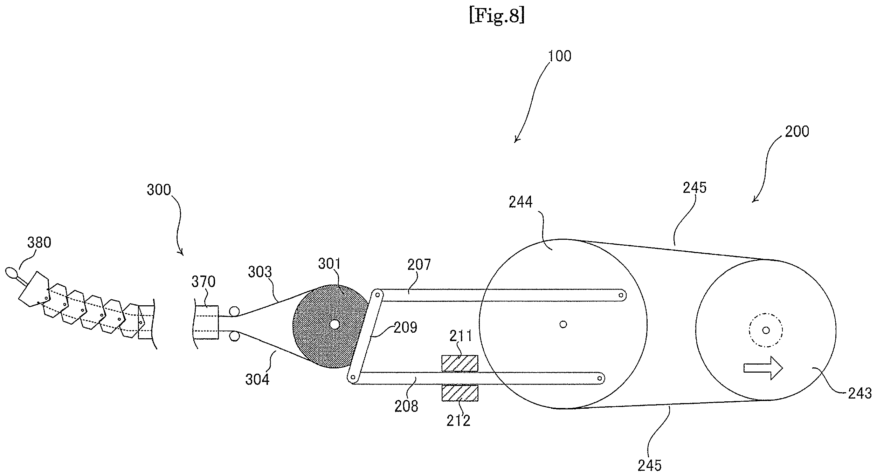

An exemplary configuration of the clutch-like mechanism in the manipulator 100 is now explained with reference to Figs. 7 and 8 illustrative of the actuation of the manipulator 100.

The manipulator 100 according to the present example differs from the previous example in that a first pulley 243 (for the second linear motion force conversion mechanism) is used instead of the gear 203 and a second pulley 244 (for the second linear motion force conversion mechanism) is used instead of the gear 205.

The first pulley 243 is attached to a shaft 221 that is rotationally driven by the motor 201.

The second pulley 244 is the same as in the former embodiment in that it rotates about a shaft 222 and is provided with the pole-form member 207 by a shaft 223 and the pole-form member 208 by a shaft 224.

An endless wire 245 (for the second linear motion force conversion mechanism) is routed around the first 243 and the second pulley 244, and the first pulley 243 is movable toward both the distal and the proximal end side by a mechanism (not shown).

As the first pulley 243 comes close to the second pulley 244, it causes the wire 245 to be relaxed, creating a disengaged state where the rotational driving force of the first pulley 243 is not transmitted to the second pulley 244.

As the first pulley 243 is released off from the second pulley 244, on the other hand, it causes the wire 245 to be tightened, creating an engaged state where the rotational driving force of the first pulley 243 is transmitted to the second pulley 244.

Fig. 7 shows that the clutch-like mechanism disengages the driving force; the first pulley 243 comes close to the second pulley 244 to keep the wire 245 relaxed. On the other hand, Fig. 8 shows that the clutch-like mechanism engages the transmission of the driving force; the first pulley 243 moves more away from the second pulley 244 than shown in Fig. 7, keeping the wire 245 tight enough for transmission of the rotational driving force from the wire 245.

The manipulator 100 described here may have such similar advantages as achieved by the manipulator 100 according to the previous example.

The second embodiment of the invention is now explained.

When the distal unit 300 is attached to the proximal unit 200 after insertion of the distal unit 300 through the channel, it is often desirable in view of operability that the end effector 380 be oriented in the direction intended by the operator. This may be achieved by allowing the seesaw member 301 in the distal unit 300 to follow forcibly the posture of the link member 209 in the proximal unit 200.

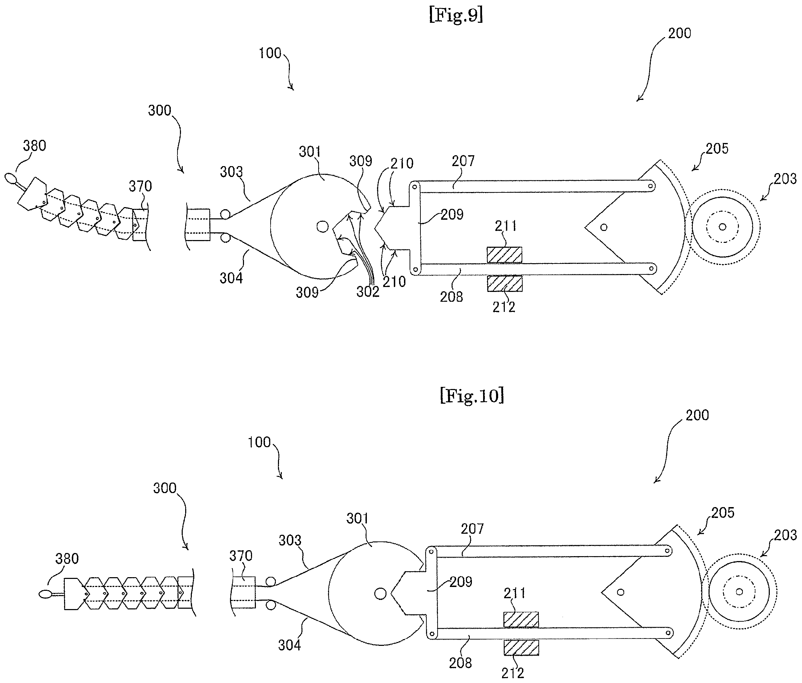

Figs. 9 and 10 are illustrative of the actuation of the manipulator 100 according to the second embodiment of the invention. The manipulator 100 according to the second embodiment differs from the manipulator 100 according to the first embodiment in that the link member 209 in the proximal unit 200 includes a convex portion in the form of a home plate, and the seesaw member 301 in the distal unit 300 includes a concave portion in the form of a home plate correspondingly.

The link member 209 includes a convex portion in the form of a home plate; there are four link member abutment portions 210 formed in a plane vertical to the sheet plane, as depicted in Fig. 9. Likewise, the seesaw member 301 includes a concave portion in the form of a home plate; there are four seesaw member contact portions 302 formed in a plane vertical to the sheet plane.

Thus, the embodiment described here is characterized in that when the distal unit 300 is attached to the proximal unit 200, the seesaw member 301 abuts on the link member 209 via a plurality of planes. This in turn causes the seesaw member 301 in the distal unit 300 to follow forcibly the posture of the link member 209 in the proximal unit 200.

Further, the concave portion in the form of a home plate in the seesaw member 301 is provided on both its side with tapered portions 309 (for the first linear motion force conversion mechanism; the contact portions) that permit for smooth fitting of the link member 209 into the seesaw member 301.

An exemplary actuation of the manipulator 100 according to the second embodiment describe here is now explained. As shown typically in Fig. 9, when the distal unit 300 is attached to the proximal unit 200 while the end effector 380 on the distal unit 300 side turns upward and the concave portion turns downward, in the form of a home plate in the seesaw member 301, the seesaw member 301 rotates in conformity with the convex portion in the form of a home plate in the link member 209 as the link member 209 in the proximal unit 200 is fitted into the seesaw member 301. Eventually, the distal unit 300 is attached to the proximal unit 200 such that the end effector 380 gets parallel with the direction toward the distal end.

As described above, the manipulator 100 according to the third embodiment described here contributes more to operability, because in the attachment of the distal unit 300 to the proximal unit 200, the end effector 380 is oriented in the direction intended by the operator.

The third embodiment of the invention is now explained. This manipulator 100 is first different from the manipulator 100 according to the first embodiment in terms of the configuration of the pole-form member 207, and the pole-form member 208.

In the manipulator 100 according the second embodiment described here, the pole-form member 207 comprises a first rack 247 (for the second linear motion force conversion mechanism, and for the second rack-and-pinion mechanism), a first rod-form member 251 (for the second power transmission assembly) extending from this first rack 247, and a rolling member 261 (for the second power transmission assembly) mounted on this first rod-form member 251.

The pole-form member 208 comprises a second rack 248 (for the second direct power conversion mechanism, and for the second rack-and-pinion mechanism), a second rod-form member 252 (for the second power transmission assembly) extending from this second rack 248, and a rolling member 262 (for the second power transmission assembly) mounted on this second rod-form member 252.

The first rack 247 is in mesh with a first pinion gear 249 (for the second linear motion force conversion mechanism, and for the second rack-and-pinion mechanism), the second rack 248 is in mesh with the first pinion gear 249, and the first pinion gear 249 is rotationally driven by the motor 201.

In the manipulator 100 according to the third embodiment described here, as the pole-form member 207 makes a displacement toward the distal end side in a direction indicated by an action arrow, it causes the pole-form member 208 to make a displacement toward the proximal end side in a direction indicated by an action arrow. Conversely, as the pole-form member 207 makes a displacement toward the proximal end side, it causes the pole-form member 208 to make a displacement toward the distal end side.

Secondly, the manipulator 100 according to the third embodiment is different from the manipulator 100 according to the first embodiment in that the seesaw member 301 is configured in the form of a rod, not in the D form, and the wires 303 and 304 are respectively fixed to an upper and a lower end of the rod-form seesaw member 301.

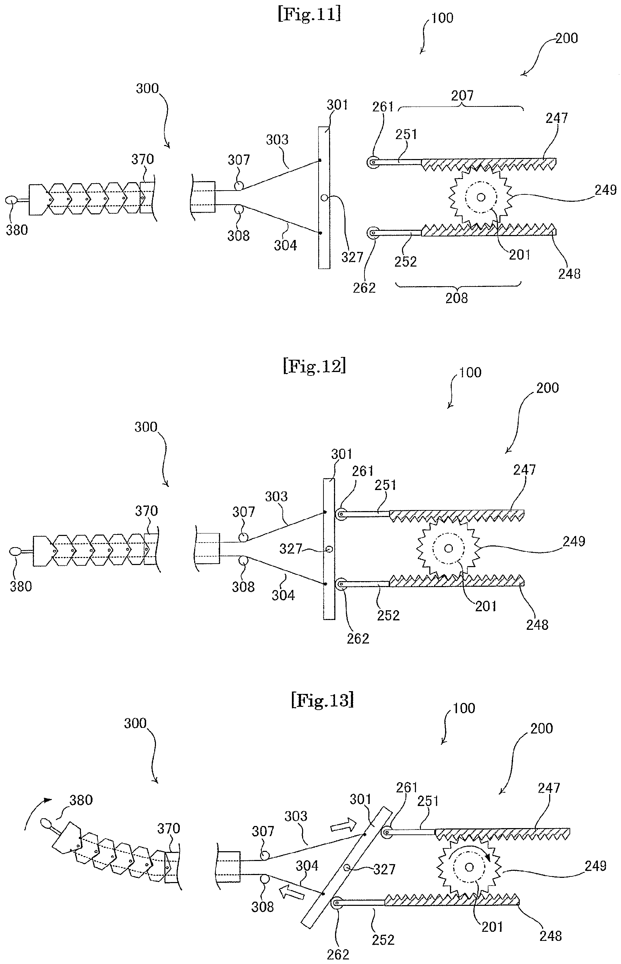

Fig. 12 is illustrative of how the distal unit 300 in the manipulator 100 according to the third embodiment of the invention is installed in place. In the manipulator 100 according to the third embodiment described here, the rolling members 261 and 262 in the proximal unit 200 abut against the seesaw member 301 for completion of installation of the distal unit 300 in place.

Fig. 13 is illustrative of the actuation of the manipulator 100 according to the third embodiment of the invention. For instance, as the motor 201 is driven to rotate the first pinion gear 249 clockwise, it causes the rolling member 261 together with the first rack 247 to make a displacement toward the proximal end side, and the rolling member 262 together with the second rack 248 to make a displacement toward the distal end side. Operatively with this, the seesaw member 301 rotates clockwise about the shaft 327 to pull the wire 303 nearer and let out the wire 304, causing an upward displacement of the end effector 380 as shown.

The manipulator 100 according to the third embodiment configured as described above, too, may have such similar advantages as achieved in the former embodiments.

Another example is now explained. The manipulator 100 according to the example is a modification to the manipulator 100 according to the third embodiment. The manipulator 100 according to the example differs from the manipulator 100 according to the third embodiment in terms of the construction of the seesaw member 301 disposed on the distal unit 300 side.

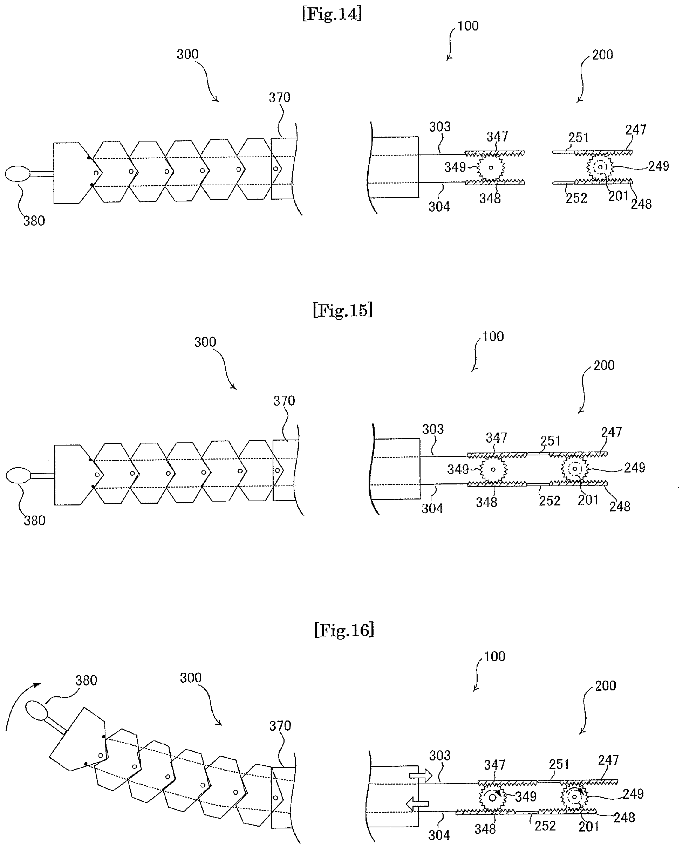

Fig. 14 is illustrative in schematic of the construction of the manipulator 100 according to the example.

In the manipulator 100 according to the example described here, the seesaw member 301 on the distal unit 300 side comprises a third rack 347 (for the first linear motion force conversion mechanism and the first rack-and-pinion mechanism) in mesh with a second pinion gear 349 (for the first linear motion force conversion mechanism and the first rack-and-pinion mechanism), and a fourth rack 348 (for the first linear motion force conversion mechanism and the first rack-and-pinion mechanism) in mesh with the second pinion gear 349.

The wire 303 is fixed at one end to the third rack 347, and the wire 304 is fixed at one end to the fourth rack 348. The driven pulleys 307 and 308 provided in the manipulator 100 according to the fifth embodiment are dispensed with, and the wires 303 and 304 are parallel with each other in the vicinity of the third 347 and the fourth rack 348. The rolling members 261 and 262 are also dispensed with.

Fig. 15 is illustrative of how the distal unit 300 in the manipulator 100 according to the present example is installed in place. In the manipulator 100 according to this example, the first rod-form member 251 in the proximal unit 200 abuts against the third rack 347 while the second rod-form member 252 abuts against the fourth rack 348 for completion of installation of the distal unit 300.

Fig. 16 is illustrative of the actuation of the manipulator 100 according to the present example. For instance, as the motor 201 is driven to rotate the first pinion gear 249 clockwise, it causes the rolling member 261 together with the first rack 247 to make a displacement toward the proximal end side and the rolling member 262 together with the second rack 248 to make a displacement toward the distal end side. Operatively with this, the third rack 347 makes a displacement toward the proximal end side, the fourth rack 348 makes a displacement toward the distal end side, and the second pinion gear 349 rotates clockwise. In turn, the wire 303 is pulled nearer by the third rack 347, and the second wire 304 is let out of the fourth rack 348, causing an upward displacement of the end effector 380 as shown.

The manipulator 100 according to the example configured as described above, too, may have such similar advantages as achieved by the former embodiments.

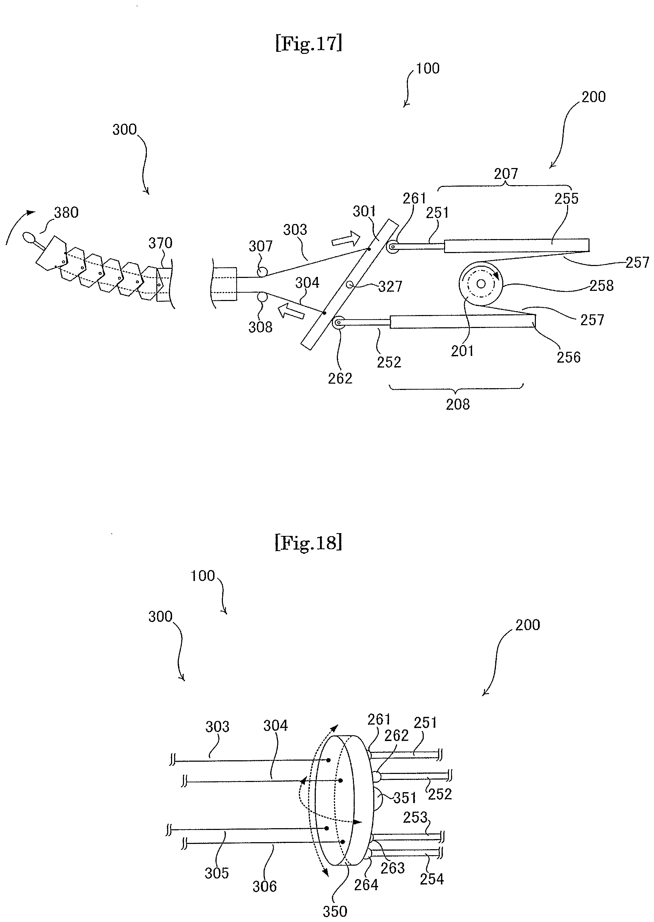

Another example is now explained. Fig. 17 is illustrative in schematic of the configuration of the manipulator 100 according to the said example. The manipulator 100 according to the example is a modification to the manipulator 100 according to the third embodiment. The manipulator 100 according to the example differs from the manipulator 100 according to the third embodiment in terms of the configuration of the pole-form members 207 and 208 in the proximal unit 200.

In the manipulator 100 according to the present example, the pole-form member 207 comprises a first rod-form member 255 (for the second power transmission assembly), and a first rod-form member 251 extending from this first rod-form member 255.

The rod-form member 208 comprises a second rod-form member 256 (for the second power transmission assembly), and a second rod-form member 252 from this second rod-form member 256.

The wire 257 (for the second linear motion force conversion mechanism) is fixed at one end to the first rod-form member 255 and at the other end to the second rod-form member 256. This wire 257 is routed around a driving pulley 258 (for the second linear motion force conversion mechanism) driven by the motor 201.

In the manipulator 100 according to the example described here, as the driving pulley 258 is rotationally driven by the motor 201, it causes the pole-form member 208 to make a displacement toward the proximal end side in a direction indicated by an action arrow when the pole-form member 207 makes a displacement toward the distal end side in a direction indicated by an action arrow. Conversely, when the pole-form member 207 makes a displacement toward the proximal end side, the pole-form member 208 makes a displacement toward the distal end side.

The manipulator 100 according to the example configured as described above, too, may have such similar advantages as achieved by the former embodiments and examples.

Another example is now explained. The manipulator 100 according to said example is a modification to the manipulator 100 according to the third embodiment.

Fig. 18 is illustrative in schematic of the configuration of the manipulator 100 according to the example. Fig. 18 shows a coupling between the proximal unit 200 and the distal unit 300.

The manipulators 100 explained so far are configured in such a way as to have a single degree of freedom. To configure a manipulator having multiple degrees of freedom, seesaw members as many as the degrees of freedom are necessary. The manipulator 100 according to the example described here is designed such that multiple degrees of freedom are operated with just one driving member. To this end, a gimbal member 350 (for the first linear motion force conversion mechanism) movable about the center of rotation 351 (for the first linear motion force conversion mechanism) is used in the distal unit 300, instead of the seesaw member 301.

In the proximal unit 200, pushing/pulling is performed on four points: rolling members 261 to 264, not on two points: rolling members 261 and 262.

According to the manipulator 100 of the example as described above, multiple degrees of freedom may be driven by a single gimbal member.

The fourth embodiment of the invention is now explained. The manipulator 100 of the fourth embodiment differs from the manipulator 100 of the first embodiment in terms of the configuration of the restriction portion used for displacements of the pole-form members 207 and 208 while they remain in a parallel state.

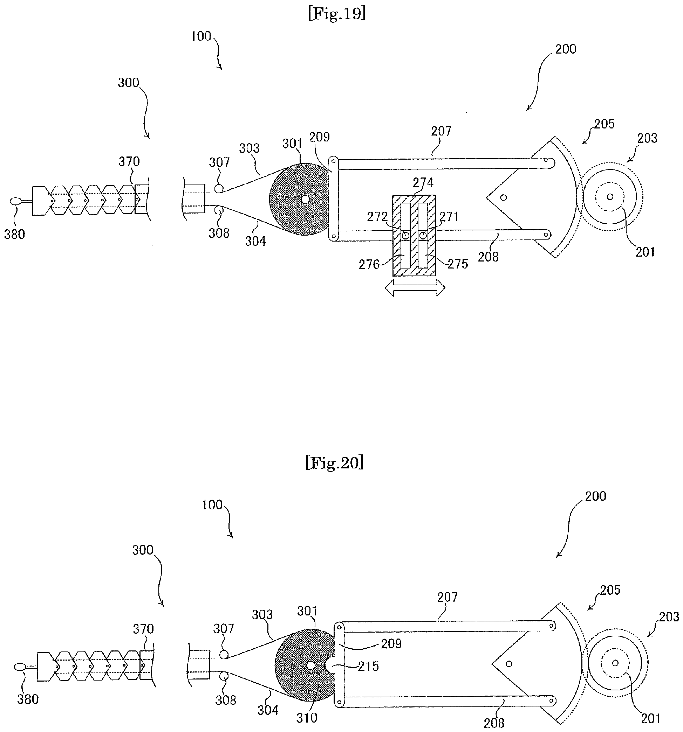

Fig. 19 is illustrative in schematic of the construction of the manipulator 100 according to the fourth embodiment of the invention. In the manipulator 100 of the fourth embodiment, a pin member 271 (for the restriction assembly) and a pin member 272 (for the restriction assembly) are provided on the pole-form member 208. These pin members are inserted through slits 275 and 276 (for the restriction assembly) in a frame member 274 (for the restriction assembly) that is horizontally moved by a mechanism (not shown), thereby restricting movement of the pole-form member 208.

The manipulator 100 of such fourth embodiment, too, may have such similar advantages as achieved by the former embodiments.

The fifth embodiment of the invention is now explained. The manipulator 100 of the fifth embodiment differs from the manipulator 100 of the first embodiment in terms of the construction of the restriction assembly used for displacements of the pole-form members 207 and 208 while they remain in a parallel state.

Fig. 20 is illustrative in schematic of the manipulator 100 according to the fifth embodiment of the invention.

In the manipulator 100 of the firth embodiment, the link member 209 in the proximal unit 200 has a convex portion 215 (for the second power transmission assembly; the abutment portion), and the seesaw member 301 in the distal unit 300 has a concave portion 310 (for the first linear motion force conversion mechanism) so that upon attachment of the distal unit 300 to the proximal unit 200, the aforesaid convex portion 215 is fitted into the aforesaid concave portion 310. Therefore, when the link member 209 fitted in the seesaw member 301 makes coordinated displacements, there is no misalignment, ensuring that movement of the pole-form members 207 and 208 is restricted.

The manipulator 100 of such fifth embodiment, too, may have such similar advantages as achieved by the former embodiments.

Another example is now explained. The manipulator 100 of said example differs from the manipulator 100 of the first embodiment in terms of the construction of the restriction assembly used for displacements of the pole-form members 207 and 208 while they remain in a parallel state.

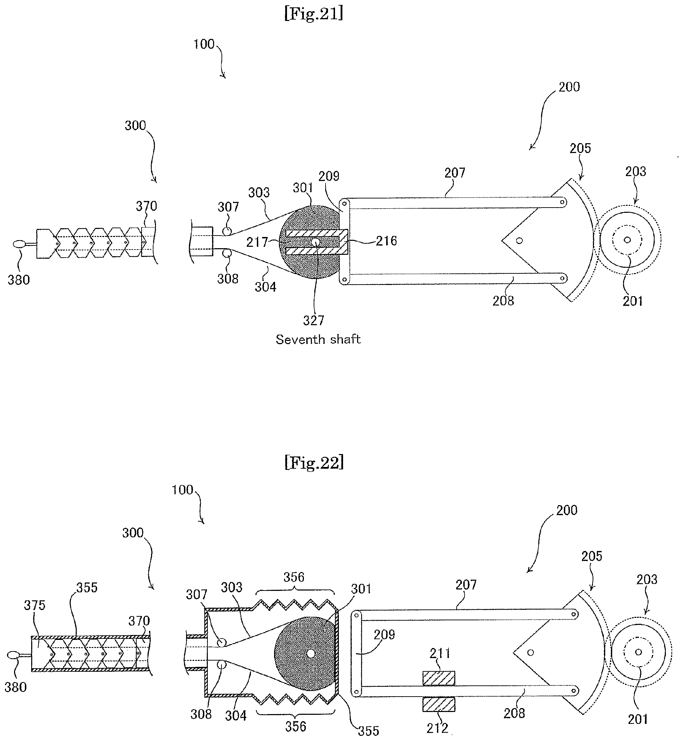

Fig. 21 is illustrative in schematic of the construction of the manipulator 100 according to the present example.

In the manipulator 100 of the example, the link member 209 is provided with a bifurcated retaining member 216 having a slit portion 217, and the distal unit 300 is attached to the proximal unit 200 by locating a shaft 327 in the aforesaid slit portion 217, ensuring that movement of the pole-form members 207 and 208 is restricted.

The manipulator 100 of such example, too, may have such similar advantages as achieved by the former embodiments and examples.

The sixth embodiment of the invention is now explained. Fig. 22 is illustrative in schematic of the construction of the manipulator 100 according to the sixth embodiment of the invention. The manipulator 100 according to the sixth embodiment differs from the manipulator 100 according to the first embodiment in that the distal unit 300 is kept watertight by a case 355 except for a part of the end effector 380 and distal end joint ring 375.

The case 355 is provided at a site for receiving the seesaw member 301 with a bellows portion 356 so that the case 355 can move following movement of the seesaw member 301.

The manipulator 100 according to such sixth embodiment may have such similar advantages as achieved in the former embodiments. In addition, keeping the interior of the distal unit 300 watertight may contribute to less cumbersome washing and shorter washing time, because washing the outside alone is all that is needed.

While the manipulators 100 according to the 1st to the sixth embodiment have been described, it is to be understood that possible manipulators 100 comprising some combinations thereof, too, are included in the category of the invention.

It is also to be noted that the aforesaid embodiments may each be applied to a so-called master/slave manipulator 1000 that is remote controlled.

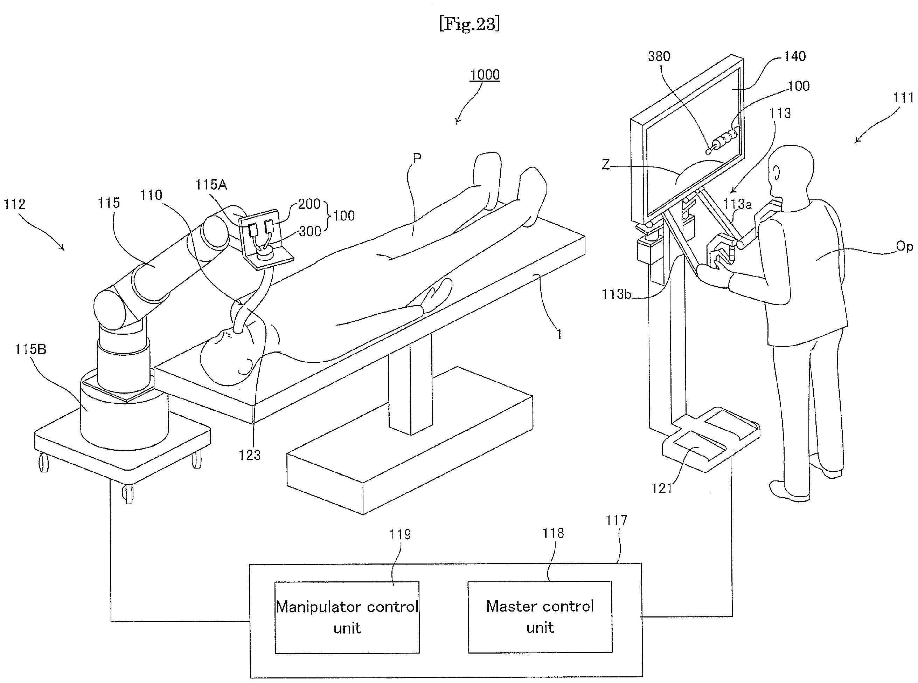

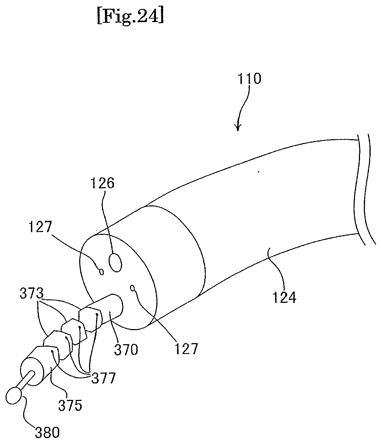

Fig. 23 is illustrative of an exemplary application of the manipulator 100 according to the invention to the master/slave manipulator system 1000, and Fig. 24 is illustrative of an endoscope device 110 in the master/slave manipulator system 1000.

The master/slave manipulator system 1000 comprises a master manipulator 111 operated by an operator Op as shown typically in Fig. 23, and a slave manipulator 112 including an endoscope device 110 shown typically in Fig. 24.

The master manipulator 111 comprises a master arm 113 in which the operator Op enters an input, a display unit 114 for displaying images, etc. taken by the endoscope device 110, a control unit 117 including a master control unit 118 for generating an operational instruction for actuation of the slave manipulator 112 and a slave control unit 119, and a control mode changeover footswitch 121.

In the example described here, the master arm 113 is an operating portion for actuation of the respective components of the slave manipulator 112 including the manipulator 100 inserted through the channel in the endoscope device 110. Although not illustrated in details, the master manipulator 111 includes a pair of master arms 113a and 113b corresponding to the right hand and left hand of the operator Op. The master arm 113 has a multi-joint structure; one master arm 113a is provided for actuation of the manipulator 100 including the end effector 380 while the other master arm 113b is done for actuation of the bending assembly 124 of the endoscope device 110.

The display unit 114 is provided for displaying images of the site to be treated, taken by the viewing optical system 126, lighting optical system 127, etc. attached to the endoscope device 110. The end effector 380 is also shown together with the site Z to be treated on the display unit 114.

The slave manipulator 112 includes a table 1 on which a patient P is lying down, a multi-joint robot 115 positioned near the table 1, and an endoscope device 110 attached to the multi-joint robot 115. Although not shown in details, the endoscope device 110 is actuated by drivers 115A, 115B mounted on the slave manipulator 112 pursuant to an operational instruction issued from the master manipulator 111.

As shown in Fig. 23, an assistant (not shown) applies proper treatments such as sterilization and anesthetization to the patient lying down on the table 1.

The operator Op instructs the assistant to introduce the insert assembly 123 from the mouth of the patient P through the body cavity. While checking on images at the display unit 114, the operator Op operates the master arm 113 to properly bend the bending portion 124 of the insert assembly 123 or actuate the end effector 380.

Even when the invention is applied to such master/slave manipulator system 1000, the advantages of the embodiments as described above may be achievable.

### APPLICABILITY TO THE INDUSTRY

The present disclosure relates to a method of controlling a medical master/slave system used while inserted through the body for applying treatments to in-vivo tissues. Endoscopic mucosal resection capable of cutting off affected sites or lesions on mucosal tissues is now expected to have a wider range of applications because there is no need for abdominal operation, resulting in limited burdens on patients and some considerable curtailments in the number of days taken until postoperative recuperation and leaving hospital. Most medical manipulators are often made up of a proximal unit for supplying driving force to the treatment tool side and a distal unit detachably mounted on the proximal unit and including a treatment tool at the distal end of a tube inserted through the patient's body. However, a problem with such manipulators is that a coupling for attachment of the distal unit to the proximal unit gets complicated. By contrast, the medical manipulator of the invention dispenses with cumbersome attachment/detachment; coupling of the proximal unit to the distal unit is all that is needed, contributing much more to operability and applicability to the industry.

### EXPLANATION OF THE REFERENCE NUMERALS

1: Table
100: Manipulator
110: Endoscope device
111: Master manipulator
112: Slave manipulator
113: Master arm
113a, 113b: Master arm
114: Display unit
115: Multi-joint robot
115A, 115B: Driver
117: Control unit
118: Master control unit
119: Slave control unit
121: Footswitch
123: Insert assembly
124: Bending portion
126: Viewing optical system
127: Lighting optical system
200: Proximal unit
201: Motor (driver)
203: Gear (for the second linear motion force conversion mechanism)
204: Gear teeth (for the second linear motion force conversion mechanism)
205: Gear (for the second linear motion force conversion mechanism)
206: Gear teeth (for the second linear motion force conversion mechanism)
207: Pole-form member (for the second power transmission assembly)
208: Pole-form member (for the second power transmission assembly)
209: Link member (for the second power transmission assembly),
210: Link member abutment portion (for the second power transmission assembly; the abutment portion)
211: First restriction member (for restriction)
212: Second restriction member (for restriction)
215: Convex portion
216: Retaining member
217: Slit
221, 222: shaft (for the second linear motion force conversion mechanism)
223, 224, 225, 226: shaft (for the second power transmission assembly),
231: Base
232: Cutout
234: Slide member
235: Through-hole
236: Upright portion
238: Biasing member
240: Pawl-form member
241: Slant portion
243: First pulley (for the second linear motion force conversion mechanism)
244: Second pulley (for the second linear motion force conversion mechanism)
245: Wire (for the second linear motion force conversion mechanism)
247: First rack (for the second linear motion force conversion mechanism; the second rack-and-pinion mechanism)
248: Second rack (for the second linear motion force conversion mechanism; the second rack-and-pinion mechanism)
249: First pinion gear (for the second linear motion conversion mechanism; the second rack-and-pinion mechanism)
251, 255: First rod-form member (for the second power transmission assembly)
252, 256: Second rod-form member (for the second power transmission assembly)
253: Third rod-form member (for the second power transmission assembly)
254: Fourth rod-form member (for the second power transmission assembly)
257: Wire (for the second linear motion force conversion mechanism)
258: Driving pulley (for the second linear motion force conversion mechanism)
261, 262, 263, 264: Rolling member (for the second power transmission assembly)
271, 272: Pin member (for restriction)
274: Frame member (for restriction)
275, 276: Slit (for restriction)
300: Distal unit
301: Seesaw member (for the first linear motion force conversion mechanism; the seesaw portion)
302: Seesaw member contact portion (for the first linear motion force conversion mechanism; the contact portion)
303, 304, 305, 306: Wire (for the first power transmission assembly)
307, 308: Driven pulley (for the first power transmission assembly)
309: Tapered portion (for the first linear motion force conversion assembly; the contact portion)
310: Concave portion (for the first linear motion force conversion mechanism)
327: Shaft (for the first linear motion force conversion mechanism)
347: Third rack (for the first linear motion force conversion mechanism; the first rack-and-pinion mechanism)
348: Fourth rack (for the first linear motion force conversion mechanism; the first rack-and-pinion mechanism)
349: Second pinion gear (for the first linear motion force conversion mechanism; the first rack-and-pinion mechanism)
350: Gimbal member (for the first linear motion force conversion mechanism)
351: Center of rotation (for the first linear motion force conversion mechanism)
355: Case
356: Bellows portion
370: Cylindrical tubular member
373: Multiple cylindrical joint rings (for the joint assembly)
375: Distal joint ring (for the joint assembly)
377: Shaft member (for the joint assembly)
380: End effector
1000: Master/slave manipulator system
Op: Operator
P: Patient
Z: Site to be treated

## Claims

1. A medical manipulator (100) comprising a distal end side and a proximal end side configured to be attachable to or detachable from each other, comprising:
a distal unit (300) including, an end effector (380) on the distal end side, a joint assembly (373, 377) located on a proximal end side of the end effector (380), and a first power transmission assembly (303, 304) for transmission in a longitudinal direction of a linear motion force for actuation of the end effector (380) or the joint assembly (373, 377);
a proximal unit (200) including, a driver (201) on the proximal end side for generating power for actuating the end effector (380) or the joint assembly (373, 377), and a second power transmission assembly (207, 208) for transmission of power generated from the driver (201) to the distal end side, and detachably mounted on the distal unit (300) at a distal end;
wherein the distal unit (300) includes a first linear motion force conversion mechanism (301, 302, 327) linked to the first power transmission assembly (303, 304) to convert power from the second power transmission assembly (207, 208) to a linear motion force upon attachment of the distal unit (300) to the proximal unit (200),
**characterized in that**
the first linear motion force conversion mechanism (301, 302, 327) includes a seesaw member (301) that is rotatable about a rotating shaft (327), and has a contact portion (302) coming in contact with the second power transmission assembly (207, 208) upon attachment of the distal unit (300) to the proximal unit (200), wherein the seesaw member (301) is connected to the first power transmission assembly (303, 304), and as a result of rotation of the seesaw member (301) by power transmitted from the second power transmission assembly (207, 208), the first power transmission assembly (303, 304) is pushed or pulled in the longitudinal direction.

2. A medical manipulator (100) as recited in claim 1, wherein the proximal unit (200) further includes a second linear motion force conversion mechanism (203, 204, 205) for converting rotational power of the driver (201) into a linear motion force, and the second power transmission assembly (207, 208) transmits the linear motion force.

3. A medical manipulator (100) as recited in claim 1, wherein the proximal unit (200) further includes a link member (209) having an abutment portion (210) in abutment on the contact portion (302) of the seesaw member (301) upon linking to a distal end of the second power transmission member (207, 208) for attachment of the distal unit (300) to the proximal unit (200).

4. A medical manipulator (100) as recited in claim 3, wherein the abutment portion (210) and the contact portion (302) are configured in such a way as to have a concave/convex relation in which the both portions are in abutment on each other.

5. A medical manipulator (100) as recited in claim 2, wherein the second power transmission member (207, 208) includes a second rack-and-pinion mechanism (247, 248, 249), a first rack (247) of the second rack-and-pinion mechanism (247, 248, 249) being in mesh with a first pinion gear (249) of the second rack-and-pinion mechanism (247, 248, 249) that is rotationally driven by the driver (201), and a second rack (248) of the second rack-and-pinion mechanism (247, 248, 249) being in mesh with the first pinion gear (249).

6. A medical manipulator (100) as recited in claim 2, which further includes a restriction portion for restricting movement of the second power transmission assembly (207, 208).

7. A medical manipulator (100) as recited in any one of claims 1 to 6, wherein the distal unit (300) is encased in such a way as to be watertight.

## Patentansprüche

1. Medizinischer Manipulator (100), umfassend eine Distalendseite und eine Proximalendseite, die dazu ausgelegt sind, aneinander angebracht oder voneinander gelöst zu werden, umfassend:
eine distale Einheit (300), umfassend einen Endeffektor (380) an der Distalendseite, eine Gelenkanordnung (373, 377), die sich auf einer Proximalendseite des Endeffektors (380) befindet, und eine erste Kraftübertragungsanordnung (303, 304) zur Übertragung einer Linearbewegungskraft zur Betätigung des Endeffektors (380) oder der Gelenkanordnung (373, 377) in einer Längsrichtung;
eine proximale Einheit (200), die einen Treiber (201) auf der Proximalendseite zum Erzeugen von Kraft zum Betätigen des Endeffektors (380) oder der Gelenkanordnung (373, 377), und eine zweite Kraftübertragungsanordnung (207, 208) zum Übertragen der von dem Treiber (201) erzeugten Kraft an die Distalendseite umfasst und abnehmbar an der distalen Einheit (300) an einem distalen Ende montiert ist;
wobei die distale Einheit (300) einen ersten Linearbewegungskraftumwandlungsmechanismus (301, 302, 327) umfasst, der mit der ersten Kraftübertragungsanordnung (303, 304) verbunden ist, um die Kraft von der zweiten Kraftübertragungsanordnung (207, 208) bei Befestigung der distalen Einheit (300) an der proximalen Einheit (200) in eine Linearbewegungskraft umzuwandeln,
**dadurch gekennzeichnet, dass**
der erste Linearbewegungskraftumwandlungsmechanismus (301, 302, 327) ein Wippelement (301) umfasst, das um eine Rotationswelle (327) rotiert werden kann, und einen Kontaktabschnitt (302) aufweist, der bei Befestigung der distalen Einheit (300) an der proximalen Einheit (200) mit der zweiten Kraftübertragungsanordnung (207, 208) in Kontakt kommt, wobei das Wippelement (301) mit der ersten Kraftübertragungsanordnung (303, 304) verbunden ist, und im Ergebnis der Rotation des Wippelements (301) durch die von der zweiten Kraftübertragungsanordnung (207, 208) übertragene Kraft die erste Kraftübertragungsanordnung (303, 304) in die Längsrichtung gedrückt oder gezogen wird.

2. Medizinischer Manipulator (100) nach Anspruch 1, wobei die proximale Einheit (200) ferner einen zweiten Linearbewegungskraftumwandlungsmechanismus (203, 204, 205) zum Umwandeln der Rotationskraft des Treibers (201) in eine Linearbewegungskraft umfasst, und die zweite Kraftübertragungsanordnung (207, 208) die Linearbewegungskraft überträgt.

3. Medizinischer Manipulator (100) nach Anspruch 1, wobei die proximale Einheit (200) ferner ein Verbindungselement (209) umfasst, das einen Angrenzungsabschnitt (210) in Angrenzung an den Kontaktabschnitt (302) des Wippelements (301) nach Verbindung mit einem distalen Ende des zweiten Kraftübertragungselements (207, 208) zur Befestigung der distalen Einheit (300) an der proximalen Einheit (200) aufweist.

4. Medizinischer Manipulator (100) nach Anspruch 3, wobei der Angrenzungsabschnitt (210) und der Kontaktabschnitt (302) derart ausgelegt sind, dass sie eine Konkav-/Konvex-Relation aufweisen, in der beide Abschnitte aneinandergrenzen.

5. Medizinischer Manipulator (100) nach Anspruch 2, wobei das zweite Kraftübertragungselement (207, 208) einen zweiten Zahnstangenmechanismus (247, 248, 249) umfasst, eine erste Zahnstange (247) des zweiten Zahnstangenmechanismus (247, 248, 249) mit einem ersten Ritzel (249) des zweiten Zahnstangenmechanismus (247, 248, 249), der von dem Treiber (201) rotationsangetrieben wird, im Eingriff ist, und eine zweite Zahnstange (248) des zweiten Zahnstangenmechanismus (247, 248, 249) mit dem ersten Ritzel (249) im Eingriff ist.

6. Medizinischer Manipulator (100) nach Anspruch 2, der ferner einen Begrenzungsabschnitt zum Begrenzen der Bewegung der zweiten Kraftübertragungsanordnung (207, 208) umfasst.

7. Medizinischer Manipulator (100) nach einem der Ansprüche 1 bis 6, wobei die distale Einheit (300) derart umhüllt ist, dass sie wasserdicht ist.

## Revendications

1. Manipulateur médical (100) comprenant un côté extrémité distale et un côté extrémité proximale configurés pour être aptes à être attachés l'un à l'autre ou détachés l'un de l'autre, comprenant :
une unité distale (300) comprenant un organe effecteur (380) sur le côté extrémité distale, un ensemble joint (373, 377) situé sur un côté extrémité proximale de l'organe effecteur (380), et un premier ensemble de transmission de puissance (303, 304) pour la transmission, dans une direction longitudinale, d'une force de mouvement linéaire pour actionnement de l'organe effecteur (380) ou de l'ensemble joint (373, 377) ;
une unité proximale (200) comprenant un élément d'entraînement (201) sur le côté extrémité proximale pour générer de la puissance pour actionner l'organe effecteur (380) ou l'ensemble joint (373, 377), et un second ensemble de transmission de puissance (207, 208) pour la transmission de puissance générée à partir de l'élément d'entraînement (201) au côté extrémité distale, et montée de manière détachable sur l'unité distale (300) au niveau d'une extrémité distale ;
l'unité distale (300) comprenant un premier mécanisme de transformation de force de mouvement linéaire (301, 302, 327) relié au premier ensemble de transmission de puissance (303, 304) pour transformer la puissance provenant du second ensemble de transmission de puissance (207, 208) en une force de mouvement linéaire lors de la fixation de l'unité distale (300) à l'unité proximale (200),
**caractérisé par le fait que**
le premier mécanisme de transformation de force de mouvement linéaire (301, 302, 327) comprend un élément de bascule (301) qui est apte à tourner autour d'un arbre tournant (327), et a une partie de contact (302) venant en contact avec le second ensemble de transmission de puissance (207, 208) lors de la fixation de l'unité distale (300) à l'unité proximale (200), l'élément de bascule (301) étant relié au premier ensemble de transmission de puissance (303, 304), et, en conséquence d'une rotation de l'élément de bascule (301) par la puissance transmise depuis le second ensemble de transmission de puissance (207, 208), le premier ensemble de transmission de puissance (303, 304) est poussé ou tiré dans la direction longitudinale.

2. Manipulateur médical (100) selon la revendication 1, dans lequel l'unité proximale (200) comprend en outre un second mécanisme de transformation de force de mouvement linéaire (203, 204, 205) pour transformer la puissance de rotation de l'élément d'entraînement (201) en une force de mouvement linéaire, et le second ensemble de transmission de puissance (207, 208) transmet la force de mouvement linéaire.

3. Manipulateur médical (100) selon la revendication 1, dans lequel l'unité proximale (200) comprend en outre un élément de liaison (209) ayant une partie de butée (210) en butée sur la partie de contact (302) de l'élément de bascule (301) lors de la liaison à une extrémité distale du second élément de transmission de puissance (207, 208) pour la fixation de l'unité distale (300) à l'unité proximale (200).

4. Manipulateur médical (100) selon la revendication 3, dans lequel la partie de butée (210) et la partie de contact (302) sont configurées de façon à avoir une relation concave/convexe dans laquelle les deux parties sont en butée l'une sur l'autre.

5. Manipulateur médical (100) selon la revendication 2, dans lequel le second élément de transmission de puissance (207, 208) comprend un second mécanisme à pignon et crémaillère (247, 248, 249), une première crémaillère (247) du second mécanisme à pignon et crémaillère (247, 248, 249) engrenant un premier pignon (249) du second mécanisme à pignon et crémaillère (247, 248, 249) qui est entraîné en rotation par l'élément d'entraînement (201), et une seconde crémaillère (248) du second mécanisme à pignon et crémaillère (247, 248, 249) engrenant le premier pignon (249).

6. Manipulateur médical (100) selon la revendication 2, qui comprend en outre une partie de limitation pour limiter un mouvement du second ensemble de transmission de puissance (207, 208).

7. Manipulateur médical (100) selon l'une quelconque des revendications 1 à 6, dans lequel l'unité distale (300) est enveloppée de façon à être étanche à l'eau.
